# DEMANDE DE BREVET EUROPEEN

(11) **EP 0 664 994 A1**
(43) Date de publication de la demande: **02.08.1995**
(21) Numéro de dépôt: 95400109.5
(22) Date de dépôt: 19.01.1995
(51) Int. Cl.: A61F 2/44, A61B 17/16, A61F 2/46, A61B 17/58

(54) **Cage intersomatique vertébrale**

(30) Priorité: 26.01.1994 FR 9400860
(71) Demandeur: BIOMAT(S.a.r.l.), F-91430 Igny (FR)
(72) Inventeur: Lahille, Michel, F-91430 Vauhallan (FR); Cottin, Philippe, F-78470 Saint Remy les Chevreuse (FR)
(74) Mandataire: Cabinet Martinet & Lapoux

(57) **Abrégé**

Une cage intersomatique (3) à insérer par voie postérieure entre deux vertébres (CV1,CV2) comprend deux branches sensiblement parallèles (31,32) pour contact avec les corps vertébraux (CV1,CV2), un pont de liaison (33) reliant des extrémités postérieures des branches (31,32), et une pièce d'écartement mobile (37) pour écarter angulairement les deux branches (31,32) après insertion de la cage (3) entre les deux vertèbres (CV1,CV2). La cage (3) permet un réglage peropératoire de l'angle de lordose entre les deux vertèbres (CV1,CV2). Un matériel ancillaire pour l'implantation de la cage (3) comprend une râpe (2) pour façonner un logement pour la cage (3) entre les deux vertébres (CV1,CV2), un porte-cage (4) pour mettre la cage (3) dans le logement, un tournevis (5) tournant une vis (36) vissée dans la pièce d'écartement (37) pour écarter les deux branches (31,32) de cage (3), et un guide (1) pour guider successivement la râpe (2), le porte-cage (4) avec la cage (3), puis le tournevis (5).

## Description

La présente invention a trait à une cage intersomatique destinée à être insérée par voie postérieure entre les corps vertébraux de deux vertèbres notamment des vertèbres lombaires, ainsi qu'à du matériel ancillaire pour l'implantation de la cage. Une telle cage permet la fusion des corps vertébraux après discectomie tout en conservant la distance intervertébrale.

Une cage intersomatique lombaire selon la technique antérieure comprend un corps cylindrique creux terminé par une extrémité hémisphérique antérieure et fermé par un bouchon postérieur vissé. Des fenêtres ou fentes longilignes sont ménagées longitudinalement dans le corps cylindrique, et/ou le corps cylindrique a une surface périphérique rugueuse moletée pour faciliter la croissance osseuse. Le corps peut être préalablement rempli de "greffons" osseux, dits "allogreffe", et le bouchon est vissé et bloqué sur le corps. Au moins une telle cage est introduite entre deux corps vertébraux à l'aide d'un matériel ancillaire approprié. Des cages de ce type sont décrites dans les W0-A-92/14423 et WO-A-89/12431 et la EP-A-0 307 241.

La cage implantée a des surfaces d'appui supérieure et inférieure contre les corps vertébraux. Ces surfaces d'appui sont des portions étroites de la surface cylindrique périphérique du corps de cage. Les dimensions de la cage sont généralement sélectionnées parmi un choix de quelques tailles pour s'adapter aux conditions anatomiques du patient. Cependant, la cage une fois choisie a des dimensions fixes. Or, il est généralement souhaitable au cours de l'intervention chirurgicale de régler la position relative des deux vertèbres qui vont être "réunies" par fusion des corps vertébraux à travers les greffons, notamment leur position angulaire dans le plan antéro-postérieur qui est appelée angle de lordose. Ce réglage peropératoire n'est pas réalisable avec une telle cage intersomatique.

La présente invention vise à remédier aux inconvénients précités en fournissant une cage intersomatique vertébrale qui permet de régler de manière peropératoire la position angulaire relative de deux vertèbres notamment lombaires dans le plan antéro-postérieur, c'est-à-dire qui contribue à imposer un angle de lordose déterminé, tout en présentant des surfaces d'appui contre les corps vertébraux plus larges que les cages connues.

A cette fin, une cage intersomatique destinée à être insérée entre deux corps vertébraux est caractérisée en ce qu'elle comprend deux branches sensiblement parallèles pour être introduites entre lesdits corps vertébraux, un pont reliant des premières extrémités des branches, et un moyen pour écarter angulairement des secondes extrémités des branches après insertion de la cage entre les corps vertèbraux.

En pratique, les premières extrémités de branche sont situées vers la voie d'accès postérieure, et le pont constitue une portion de liaison de la cage monolithique avec les branches. Les secondes extrémités de branche sont dirigées vers la face antérieure des corps vertébraux. Le chirurgien choisit l'angle de lordose le plus approprié aux besoins anatomo-physiologiques du patient grâce à l'écartement angulaire entre les secondes extrémités des branches.

Une coopération entre le moyen pour écarter et les branches a été recherchée de manière à commander l'écartement souhaité par l'extrémité distale postérieure de la cage, seule accessible après implantation de la cage par voie postérieure. Les caractéristiques suivantes de la cage selon l'invention contribuent à cet objectif.

Les secondes extrémités des branches ont des surfaces d'appui en regard qui forment les côtés d'un vé dont le sommet est orienté vers le pont et qui sont en contact glissant avec le moyen pour écarter. Une fente s'étend depuis les secondes extrémités des branches, est située entre les branches et est traversée longitudinalement par le moyen pour écarter. La fente est de préférence terminée près du pont par un élargissement. L'élargissement de fente aux extrémités postérieures des branches augmente la flexibilité des branches soumises à écartement pour choisir l'angle de lordose le plus approprié.

Selon une réalisation préférée, le moyen pour écarter comprend une vis comprenant une tête en appui contre le pont et une tige filetée traversant le pont et passant entre les branches, et une pièce d'écartement en contact avec des surfaces d'appui en regard des secondes extrémités de branche et vissée à la tige filetée de la vis.

De préférence, l'extrémité de la tige filetée de la vis comprend un moyen d'arrêt, tel que rondelle ou collet pour maintenir la pièce d'écartement sur la tige filetée et ainsi éviter un démontage indésirable de la vis et de la pièce d'écartement par un dévissage excessif.

La tête de vis est par exemple une tête fendue simplement ou en croix, ou une tête à six pans creux qui permet la manoeuvre de la vis par un tournevis approprié.

D'autre part, une face du pont opposée aux branches comprend un logement circulaire par exemple à section en queue d'aronde, tronconique ou en té, parallèle aux branches, c'est-à-dire longitudinal à la cage, et traversé perpendiculairement par une rainure pour introduire une extrémité correspondante d'un porte-cage et ainsi relier de manière démontable la cage au porte-cage lors de l'implantation de la cage.

Dans un autre aspect de l'invention, la cage est sensiblement parallélépipédique et offre ainsi des surfaces d'appui planes sur les corps vertébraux.

Ces surfaces d'appui sont des faces externes des branches comprenant des moyens d'ancrage transversaux pour ancrer les branches dans les corps vertébraux avec lesquels les faces externes sont en contact intime. De préférence, les moyens d'ancrage sont répartis en des dentures plus petites aux premières extrémités de branche et des dentures plus grandes aux secondes extrémités de branche afin de faciliter une "articulation" des branches à proximité du pont et un retrait éventuel de la cage après que les branches soient rapprochées jusqu'à ce qu'elles soient sensiblement parallèles.

Chacune des branches comprend un trou oblong s'étendant sensiblement entre les première et seconde extrémités de la branche. Le trou oblong peut contenir des fragments osseux pour une bonne reprise osseuse entre les corps vertèbraux.

L'invention concerne également un guide pour guider un objet, notamment une cage, ou bien une râpe, un porte-cage ou tournevis comme on le verra ci-après, vers un milieu osseux tel qu'espace intervertébral entre deux corps vertébraux. Le guide comprend un conduit longitudinal de guidage dans lequel la cage peut glisser longitudinalement, et deux pointes saillantes devant une première extrémité du conduit de guidage pour être plantées dans le milieu osseux. Grâce aux pointes, la première extrémité du conduit de guidage est liée au milieu osseux vertébral après discectomie de disque. Le chirurgien a ainsi un accès privilégié à une zone bien déterminée de l'espace intervertébral, ce qui facilite l'intervention sur cette zone tout en protégeant le milieu environnant dans le corps humain.

Le conduit de guidage peut être cylindrique et comprendre à une seconde extrémité un moyen pour positionner en rotation la cage introduite par la seconde extrémité. Le moyen pour positionner comprend un ergot saillant radialement vers l'intérieur du conduit du guidage et pouvant coopérer avec une rainure longitudinale externe de la râpe, ou du porte-cage ou du tournevis. A des fins de retrait du guide du corps humain, le guide comprend une surface d'appui située sensiblement à une seconde extrémité du conduit de guidage, sensiblement perpendiculaire au conduit de guidage, et dirigée vers la première extrémité. Au moyen d'un outil de retrait, tel qu'une enclume cylindrique avec une rainure pour recevoir une portion de guide intermédiaire entre les première et seconde extrémités, du conduit de guide, les pointes sont retirées du milieu osseux par des poussées appliquées sur la surface d'appui du guide à travers l'outil de retrait.

L'invention a également trait à une râpe pour façonner un logement, notamment entre les corps vertébraux de deux vertèbres pour y insérer la cage intersomatique selon l'invention. La râpe comprend une tête de râpe ayant une section transversale analogue à celle de la section transversale de la cage perpendiculaire aux branches et un conduit de râpe longitudinal débouchant en bout de la tête de râpe. Au moins une face longitudinale de la tête de râpe est munie de dents et d'encoches. Les encoches constituent des trous localisés entre les dents et débouchant dans le conduit de râpe. Le conduit de râpe recueille ainsi des fragments osseux pour confectionner ultérieurement des greffons osseux.

Les encoches et donc des bords d'attaque des dents sont de préférence inclinés d'environ 30 à 45° par rapport à la face longitudinale de la tête de râpe afin de râper efficacement le milieu osseux, tel que les corps vertébraux.

La râpe est introduite dans le guide selon l'invention et comprend une rainure longitudinale pour coopérer avec le moyen pour positionner en rotation, tel que l'ergot, dans le guide. La cage est ainsi convenablement positionnée avec des branches sensiblement parallèles aux deux corps vertébraux avant que la cage soit insérée dans le logement usiné par la râpe et situé entre les deux corps vertébraux.

La râpe comprend en outre une butée externe localisée à une autre extrémité de la râpe opposée à la tête de râpe pour buter contre la surface porteuse à la seconde extrémité du guide et ainsi limiter la course en translation de la râpe introduite dans le guide et la profondeur du logement à usiner dans le milieu osseux.

L'invention concerne également un porte-cage pour liaison amovible avec une cage selon l'invention. Le porte-cage comprend un tenon d'extrémité ayant une section transversale sensiblement complémentaire du logement circulaire dans le pont de cage et susceptible de traverser librement la rainure dans le pont de cage.

Le porte-cage portant en bout la cage par le tenon est introduit à glissement dans le guide. Le porte-cage comprend en outre un corps longiligne ayant une rainure longitudinale pour coopérer avec le moyen, tel qu'ergot, pour positionner en rotation afin de positionner en rotation le porte-cage dans le conduit de guide, et un dégagement situé à une extrémité de la rainure opposée au tenon. Lorsqu'il est en regard du moyen pour positionner en rotation, le dégagement autorise une rotation axiale du porte-cage dans le conduit de guidage.

Enfin, l'invention concerne un tournevis pour tourner à distance la vis dans la cage afin d'écarter les branches de la cage. Ce tournevis comprend un corps pouvant être introduit à glissement dans le conduit de guidage du guide conforme à l'invention. Le corps de tournevis a à une extrémité un embout à section complémentaire d'un moyen dans la tête de vis afin de tourner la tête de vis, et à une autre extrémité une poignée de manoeuvre qui peut buter contre la surface porteuse à la seconde extrémité du guide.

D'autres caractéristiques et avantages de la présente invention apparaîtront plus clairement à la lecture de la description suivante de plusieurs réalisations préférées de l'invention en référence aux dessins annexés correspondants dans lesquels :
- la figure 1 est une vue longitudinale en coupe partielle d'un guide selon l'invention ;
- la figure 2 est une vue de face antérieure proximale du guide ;
- la figure 3 est une vue longitudinale de côté partiellement en coupe axiale d'une râpe selon l'invention ;
- la figure 4 est une vue longitudinale de dessus de la râpe ;
- la figure 5 est une vue de face antérieure proximale de la râpe ;
- la figure 6 est une vue en perspective d'une cage selon l'invention ;
- la figure 7 est une vue longitudinale de côté partiellement en coupe axiale de la cage sans moyen pour écarter des branches de la cage;
- la figure 8 est une vue de face distale postérieure de la cage ;
- la figure 9 est une vue de face proximale antérieure de la cage ;
- la figure 10 est une vue longitudinale d'un porte-cage selon l'invention ;
- la figure 11 est une vue en bout proximal antérieur du porte-cage ;
- la figure 12 est une vue longitudinale partiellement en coupe du porte-cage lié à la cage et inséré dans le guide ;
- la figure 13 est une vue longitudinale d'un tournevis selon l'invention ;
- la figure 14 est une vue longitudinale partiellement en coupe d'un tournevis coopérant avec la cage et inséré dans le guide ;
- la figure 15 est une vue de côté de la cage selon l'invention avec des branches écartées ;
- la figure 16 est une vue longitudinale du guide et d'une enclume pour retirer le guide selon l'invention ; et
- la figure 17 est une section transversale prise le long de la ligne XVII-XVII de la figure 16.

Le matériel ancillaire et la cage intersomatique selon l'invention sont décrits en suivant sensiblement l'ordre chronologique de leur utilisation par un chirurgien lors d'une intervention chirurgicale par voie postérieure sur le rachis d'un patient. Le matériel ancillaire et la cage sont réalisés en un matériau biocompatible, par exemple titane ou alliage à base de titane.

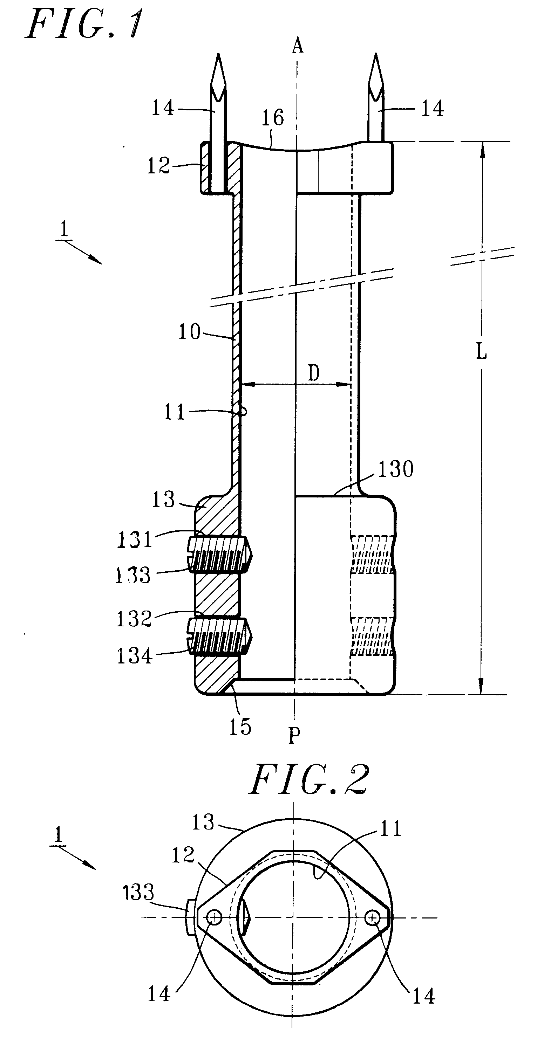

En référence aux figures 1 et 2, un guide 1 comprend un corps longiligne 10 percé longitudinalement par un conduit 11 et terminé par des première et seconde extrémités 12 et 13. La longueur L du guide 1 est d'environ 20 à 25 cm.

Le conduit 11 est cylindrique et a un diamètre constant D le long d'un axe longitudinal postérieur-antérieur P-A. La première extrémité 12, dite extrémité proximale antérieure, est une collerette destinée à entrer en contact avec le milieu osseux de corps vertébraux. La collerette 12 est sensiblement ovale, losangique ou hexagonale selon la figure 2. Deux pointes d'ancrage identiques 14 saillent depuis deux sommets de la collerette diamétralement opposés, vers l'extérieur du guide et parallèlement à l'axe P-A. Les pointes 14 sont symétriques par rapport à l'axe P-A et ont une longueur d'environ 1 cm. La seconde extrémité 13 est une extrémité distale dans laquelle peuvent être introduites différentes pièces, notamment une râpe et une cage selon l'invention. L'extrémité postérieure distale 13 est un épaulement cylindrique d'une longueur d'environ 1 à 2 cm qui forme une surface d'appui annulaire 130 à la jonction avec le corps 10. Deux trous taraudés 131 et 132 radiaux dans l'extrémité 13 sont axés dans un plan diamétral commun au conduit 11 et reçoivent deux ergots de guidage filetés de type vis-pointeau sans tête 133 et 134. La longueur des ergots est telle qu'une fois vissés dans les trous 131 et 132, les ergots saillent radialement dans le conduit 11 sur une longueur de 1 à 2 mm. Les ergots sont espacés parallèlement d'environ 5 mm. Comme il apparaîtra dans la suite, les ergots 133 et 134 constituent des moyens de positionnement en rotation de différentes pièces guidées par le guide 1.

Selon diverses réalisations, le positionnement en rotation est réalisé par un seul ergot saillant dans le conduit 11 ou par deux ergots symétriques par rapport à l'axe P-A ou encore par quatre ergots symétriques deux à deux par rapport à l'axe P-A. Selon une autre variante, le conduit 11 n'est pas cylindrique mais a une section carrée ou rectangulaire adaptée aux pièces à guider par le guide 1.

Le conduit 11 débouche par une fraisure postérieure 15 à l'extrémité distale 13 et par un dégagement cylindrique antérieur 16 s'étendant perpendiculairement à l'axe P-A à l'extrémité proximale 12. La fraisure 15 et le dégagement 16 facilitent l'insertion et la sortie des différentes pièces guidées par le guide 1.

Le guide 1 est mis en place après discectomie par voie postérieure pour enlever un disque déficient entre deux vertèbres. Le chirurgien applique l'extrémité proximale antérieure 12 contre les deux vertèbres jusqu'à planter les pointes 14 dans un corps vertébral supérieur et un corps vertébral inférieur séparés par le disque déficient de manière à assurer l'ancrage peropératoire du guide 1 dans les vertèbres et atteindre l'espace intervertébral par le conduit 11 tout en protégeant la dure-mère et les racines vertébrales.

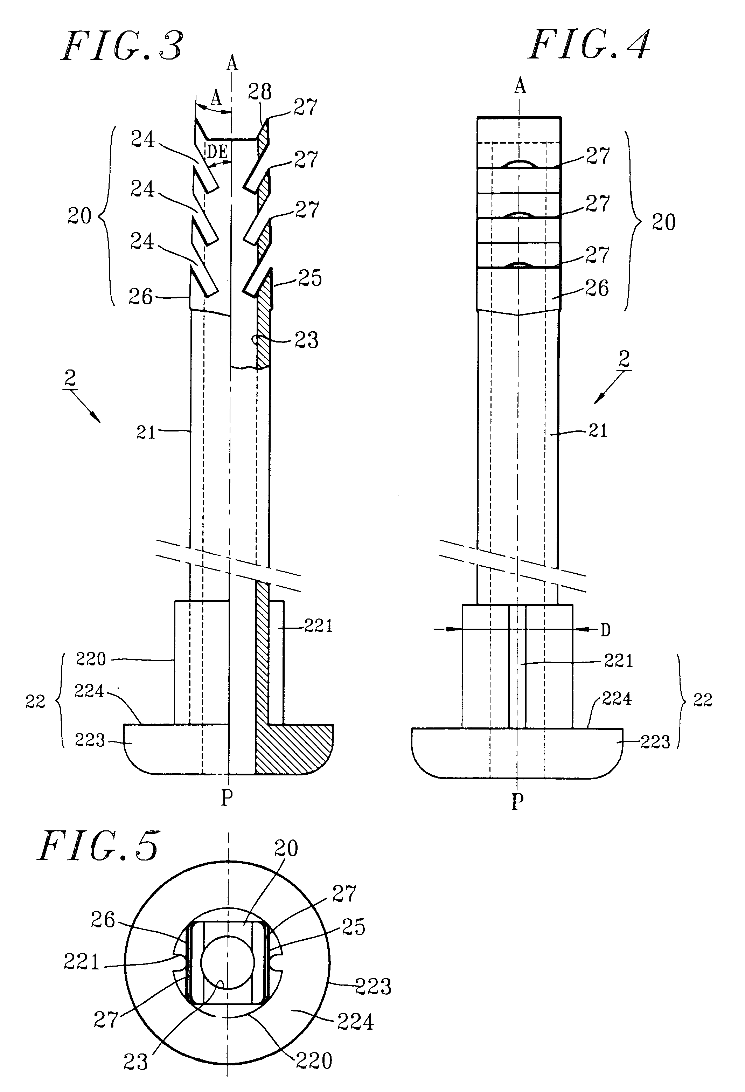

Selon les figures 3, 4 et 5, une râpe 2 pour façonner un logement entre deux corps vertébraux comprend, suivant un axe longitudinal P-A, une tête proximale antérieure 20 prolongée par un long corps 21 terminé en une extrémité de préhension postérieure 22.

La tête 20 a une longueur de l'ordre de 2 à 3 cm. La forme générale de la tête 20 est celle d'une pyramide tronquée à section rectangulaire convergeant en direction antérieure avec un angle A de quelques degrés par rapport à l'axe longitudinal P-A. La grande base rectangulaire ou carrée de la forme pyramidale est sensiblement inscrite dans la section transversale D du conduit 11. Les dimensions de la tête 20 sont sensiblement analogues à celles d'une cage intersomatique selon l'invention.

La tête, le corps et l'extrémité de préhension de la râpe 2 sont percés suivant l'axe P-A par un conduit cylindrique 23 qui débouche au bout de la tête 20 et de l'extrémité 22. En variante, seulement la tête 20, ou la tête et seulement une partie du corps 21 adjacente à la tête sont percées par le conduit 23 qui est alors borgne.

La tête 20 comprend des encoches obliques 24 sur des faces supérieure et inférieure 25 et 26 de la tête 20. Les encoches 24 sont inclinées d'un angle d'environ 30 à 45 degrés par rapport aux surfaces supérieure et inférieure de la tête et débouchent dans le conduit cylindrique 23. A titre d'exemple, selon la réalisation illustrée, trois encoches 24 sont régulièrement réparties sur chacune des deux faces supérieure et inférieure 25 et 26. Les encoches forment entr'elles des dents parallèles acérées 27 dirigées vers l'extrémité antérieure proximale de la tête 20 qui comprend un évidement 28 à section trapézoïdale formant une dent supplémentaire 27 sur chaque face 25, 26.

Le corps longiligne 21 a une section carrée dont le côté a une longueur inférieure ou égale à celle de la grande base rectangulaire de la tête pyramidale 20. En variante, le corps 21 est cylindrique de diamètre inférieur ou égal au diamètre D du conduit 11.

L'extrémité de préhension 22 comprend une portion cylindrique de guidage 220 dont le diamètre correspond au diamètre D du conduit 11 du guide 1. La portion 220 a une longueur de l'ordre de 3 à 4 centimètres et comprend une rainure longitudinale 221 propre à recevoir les extrémités des ergots de positionnement 133 et 134 du guide 1. En variante, la portion 220 comprend une seconde rainure symétrique de la rainure 221 par rapport à l'axe P-A. La seconde rainure coopère avec des ergots du guide 1 symétriques des ergots 133 et 134 par rapport à l'axe P-A. Les rainures 221 permettent de positionner la râpe 2 indifféremment suivant deux positions dans le guide 1, que celui-ci ait deux ou quatre ergots suivant les diverses réalisations d'extrémité 13 décrites en référence aux figures 1 et 2.

L'extrémité 22 se termine par une butée circulaire 223 qui forme avec la portion 220 un épaulement 224 et qui offre un diamètre plus grand que le diamètre D du conduit 11. La longueur comprise entre l'épaulement 224 et la jonction du corps 21 et de la tête 20 est sensiblement égale à la longueur L du guide 1.

L'utilisation de la râpe 2 est la suivante. Le guide 1 étant ancré dans deux corps vertébraux comme précédemment décrit, le chirurgien introduit la tête de râpe 20 dans le guide 1 par l'extrémité d'introduction distale 13 et fait coulisser la râpe dans le guide. Lorsque la tête 20 arrive près de l'extrémité proximale 12 du guide, la rainure 221 reçoit les ergots de guidage 133 et 134 de sorte que la translation de la râpe est alors guidée par les ergots qui empêchent toute rotation indésirable de la râpe dans le guide.

Le chirurgien pousse progressivement en va-et-vient la râpe 2 dans le guide 1. Les faces supérieure et inférieure 25 et 26 de la râpe grattent respectivement la face inférieure du corps vertébral supérieur et la face supérieure du corps vertébral inférieur pour façonner un logement de dimensions correspondant à celles de la râpe entre les deux corps vertébraux. Le râpage est effectué jusqu'à ce que la surface d'appui 224 de la butée 223 vienne en contact avec la face de l'extrémité 13 du guide. La tête de râpe 20 saille alors de l'extrémité antérieure proximale 12 du guide dans l'espace intervertébral.

La longueur de la râpe est telle que lorsque la râpe est insérée à fond dans le guide, la tête de râpe 20 saille d'une longueur prédéterminée de l'extrémité 12 du guide, typiquement comprise entre 2 et 3 cm, afin de ne pas endommager le rachis, notamment la dure-mère et les racines rachidiennes, par une pénétration trop profonde de la râpe entre les deux corps vertèbraux.

Lors de la formation du logement, les dents 27 des faces supérieure et inférieure de la râpe façonnent les os, et les encoches 24 recueillent la râpure sous forme de fragments osseux qui sont quasiment pulvérulents et qui sont récupérés dans le conduit 23. Après avoir façonné le milieu osseux pour former le logement, le chirurgien retire la râpe et dispose ainsi des fragments osseux pour constituer un greffon au cours de l'intervention chirurgicale.

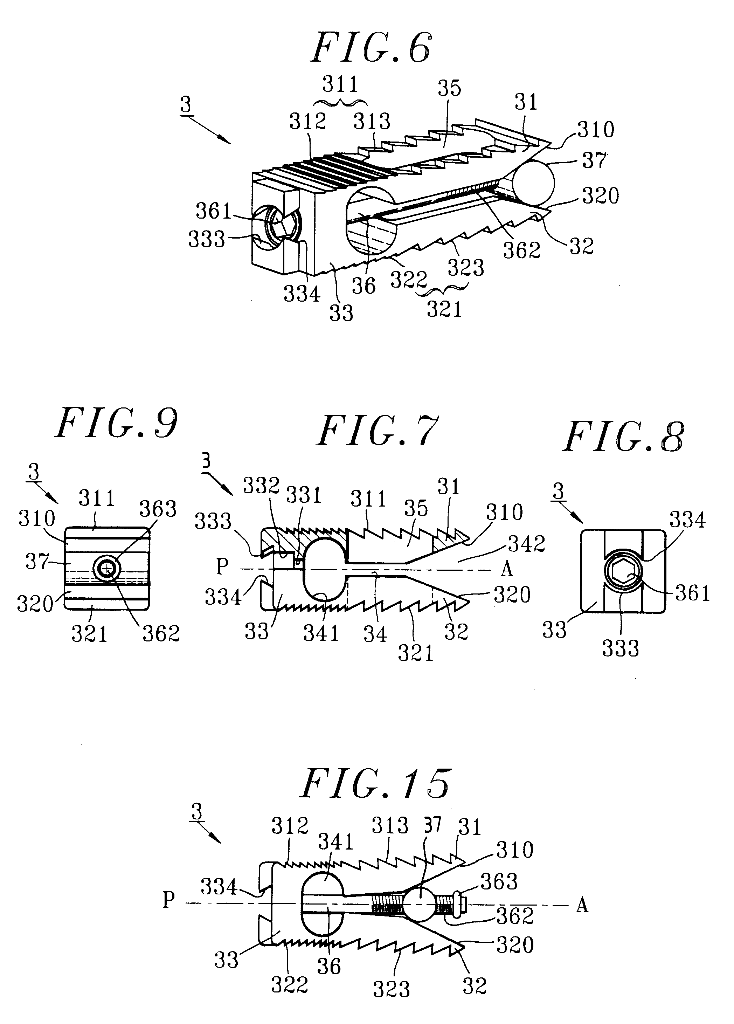

En référence aux figures 6 à 9, une cage intervertébrale 3 selon l'invention est de forme sensiblement parallélépipédique à profil longitudinal en U symétrique par rapport à un plan longitudinal postéro-antérieur de trace P-A et perpendiculaire au plan de la figure 7.

Deux branches longitudinales 31 et 32 de la cage sont reliées par un pont de liaison 33 transversal à l'axe P-A. Un trou lisse traversier axial 331 à travers le pont de liaison comprend un lamage 332 formant une surface d'appui pour une tête de vis. Un logement tronconique en queue d'aronde 333 dans la face extérieure sensiblement carrée du pont converge vers la direction postérieure au-dessus du lamage 332 et est fendu par une rainure transversale 334.

Une fente longitudinale 34 sépare les deux branches 31 et 32. La fente 34 est terminée sous le pont 33 par un élargissement oblong 341 ou sensiblement cylindrique, perpendiculaire à l'axe P-A. A l'extrémité proximale antérieure de la cage, la fente 34 débouche en s'évasant par une encoche en vé 342 dont le sommet est orienté vers le pont de liaison 33 et dont les côtés forment des surfaces d'appui 310 et 320 obliques par rapport à l'axe P-A respectivement sur les branches 31 et 32.

Les faces externes 311 et 321 des branches supérieure et inférieure 31 et 32 présentent des moyens d'ancrage en dent de scie. Chaque face 311, 321 comprend une première denture transversale 312, 322 s'étendant sensiblement depuis l'extrémité postérieure distale de la cage jusque sensiblement au droit du côté proximal de l'élargissement 341, et une seconde denture transversale 313, 323 qui a des dents plus hautes et longues que celles de la première denture et qui s'étend sur le reste de la cage jusqu'à l'extrémité proximale antérieure de celle-ci, au niveau de la fente 34 et de l'encoche 342.

En variante, la face 311, 321 ne comprend qu'une denture, ou bien au moins l'une des deux dentures sur la face 311, 321 est remplacée par des picots de préférence coniques pointus.

Chacune des deux branches 31 et 32 est en outre percée par une lumière oblongue 35 débouchant dans la fente 34.

Une vis 36 s'étend longitudinalement le long de l'axe P-A. Une tête à six pans creux 361 de la vis est en appui dans le lamage 332. Une tige de vis à extrémité filetée 362 traverse librement le trou lisse 331, l'élargissement 341 et la fente 34. L'extrémité antérieure de la vis 35 est sensiblement à l'aplomb, ou légèrement en retrait, de l'extrémité antérieure de la cage comprenant les surfaces d'appui obliques 310 et 320. Un petit galet cylindrique d'écartement 37 comprend à mi-longueur un trou diamétral taraudé dans lequel est vissée la tige filetée 362 de la vis 36 afin que la surface cylindrique du galet soit en contact glissant et bute symétriquement sur les surfaces d'appui obliques 310 et 320. En variante, le galet d'écartement 37 n'est pas cylindrique mais est un élément qui a un profil transversal sensiblement trapézoïdal ayant des côtés offrant des surfaces plus grandes en contact avec les surfaces obliques 310 et 320.

L'extrémité de la tige filetée 362 de la vis 36 comprend une rondelle d'arrêt 363, par exemple soudée à l'argon sur la tige 362, pour empêcher le démontage du galet ou élément d'écartement 37 par un dévissage excessif de la vis 36. En variante, la rondelle 363 est remplacée par une collerette obtenue à l'extrémité de la tige filetée 362 par écrasement et refoulement de métal après le vissage de la tige filetée dans le trou du galet ou élément d'écartement.

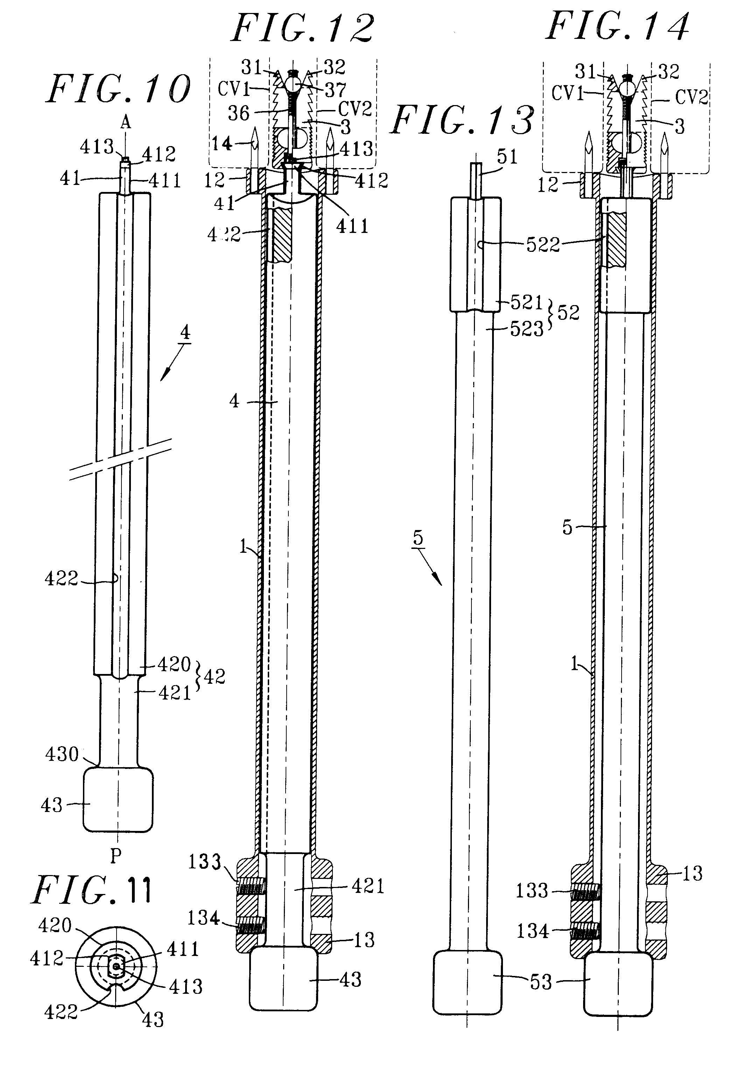

En référence aux figures 10 et 11, un porte-cage 4 est un long corps 42 s'étendant suivant la direction de l'axe postéro-antérieur P-A et terminé par une extrémité de liaison 41 avec la cage 3 et par une tête de préhension 43. L'extrémité de liaison 41 comporte en bout un tenon sensiblement rectangulaire en queue d'aronde 411 à petits chants circulaires 412 complémentaires du logement circulaire en queue d'aronde 333 dans le pont 33 de la cage 3. Le tenon 411 a une largeur inférieure à celle de la rainure transversale d'introduction 334 afin que le tenon 41 soit insérable dans la rainure 334 par le dessus de celle-ci, et a une longueur correspondant sensiblement au "diamètre" du logement afin que, après introduction dans la rainure 334, le tenon 411 soit tourné sur lui-même d'environ 90° pour le bloquer en translation axiale dans le logement circulaire en queue d'aronde contenante 333. De préférence un ergot 413 saille longitudinalement au bout de l'extrémité de liaison 41 pour le loger dans la tête creuse de vis 361 afin de positionner plus facilement le porte-cage 4 relativement à la cage 3.

Le corps longiligne 42 comprend une première portion cylindrique 420 de diamètre correspondant au diamètre D du conduit 11 du guide 1. La portion 420 est longue et s'étend depuis l'extrémité antérieure 41 jusqu'à une seconde portion cylindrique 421 formant un dégagement de diamètre inférieur à celui de la portion 420 et s'étendant jusqu'à la tête de préhension 43. Dans la longue portion 420 est ménagée une rainure longitudinale 422, ou en variante sont ménagées deux rainures longitudinales symétriques par rapport à l'axe P-A. La section transversale de la rainure longitudinale 422 est adaptée au diamètre des ergots de positionnement 133 et 134 du guide 1.

La tête de préhension 43 a un diamètre supérieur au diamètre D du conduit 11 du guide 1 et forme une butée d'arrêt 430. La longueur du porte-cage entre l'extrémité de liaison 41 à relier à la cage 3 et la butée d'arrêt 430 de la tête de préhension est sensiblement égale à la longueur L du guide 1, comme montré à la figure 12.

La cage est montée de manière amovible à l'extrémité 41 du porte-cage en insérant axialement le tenon 411 dans la rainure 334 et en tournant le porte-cage de 90° pour bloquer le tenon 411 dans le logement circulaire en queue d'aronde 333. La cage est alors liée au porte-cage et les axes longitudinaux P-A de la cage 3 et du porte-cage 4 sont confondus.

Selon la figure 12, l'ensemble porte-cage et cage 4 et 3 est introduit dans le guide 1 par l'extrémité distale d'introduction 13, puis est guidé et bloqué en rotation axiale par le glissement de la rainure 422 devant les ergots 133 et 134.

Après que la tête de préhension 43 du porte-cage arrive en butée sur l'extrémité 13 du guide, la cage 3 saille de l'extrémité proximale antérieure 12 du guide et est située dans le logement intervertébral qui est façonné par la râpe 2 entre les corps vertébraux inférieur et supérieur désignés par CV1 et CV2 dans la figure 12 et dont les dimensions correspondent sensiblement à celles de la cage. Les faces externes supérieure et inférieure 311 et 321 de la cage sont en contact intime avec les corps vertébraux CV1 et CV2 respectivement. Les dentures 312, 313 et 322, 323 ancrent la cage entre les corps vertébraux CV1 et CV2, et les lumières 35 favorisent une croissance osseuse intervertébrale. Lorsque la cage 3 est située dans le logement intervertébral, la portion postérieure cylindrique 421 du porte-cage est en regard des ergots de positionnement 133 et 134. Le rayon de la portion cylindrique 421 est inférieur à la distance entre l'axe P-A et les ergots 133 et 134. Le porte-cage 4 est par conséquent libre en rotation axiale dans le guide 1 alors que la cage 3 demeure bloquée en rotation entre les corps vertébraux CV1 et CV2. En saisissant le porte-cage 4 par la tête de préhension 43, le chirurgien fait alors tourner de 90° le porte-cage 4 pour dissocier le tenon 411 du logement circulaire en queue d'aronde 333 en alignant le tenon 411 dans la rainure 334. La cage implantée 3 est dissociée du porte-cage par le chirurgien qui retire ensuite le porte-cage 4 en repositionnant la rainure 422 en regard des ergots 133 et 134 et en faisant coulisser le porte-cage 4 dans le conduit 11. Selon d'autres variantes, des sections contenue et contenante du logement 333 et du tenon 41, par exemple en té ou rainure hélicoïdale et tenon du type verrouillage à baïonnette, autres que celles en queue d'aronde peuvent être choisies.

En référence aux figures 13 et 14, un tournevis 5 comprend un embout proximal 51 en forme de tige à section hexagonale correspondant à la tête à six pans creux 361 de la vis 36 dans la cage 3. L'embout 51 est en en-tête d'une longue tige 52 terminée par une poignée de manoeuvre 53.

La tige 52 avec l'embout 51 a une longueur sensiblement supérieure à celle du conduit 11 du guide et comprend une portion de guidage proximale 521 de laquelle saille axialement l'embout hexagonal 51 et qui a un diamètre correspondant au diamètre D du conduit 11, pour guider précisément l'embout de tournevis 51 dans la tête de vis de cage 361. La portion de guidage 521 comprend au moins une rainure longitudinale 522 correspondant aux ergots 133 et 134 du guide 1. La tige 52 comprend une portion cylindrique intermédiaire 523 plus longue et de diamètre plus faible, s'étendant entre la portion de guidage 521 et la poignée 53. L'extrémité distale postérieure de la portion intermédiaire 523 est en regard des ergots 133 et 134 lorsque le tournevis 5 est inséré dans le guide 1. La portion 523 a un diamètre comme celui de la portion cylindrique distale 421 du porte-cage 4 pour permettre la libre rotation du tournevis 5 dans le conduit 11 du guide sans que les ergots de positionnement 133 et 134 du guide y fassent obstacle.

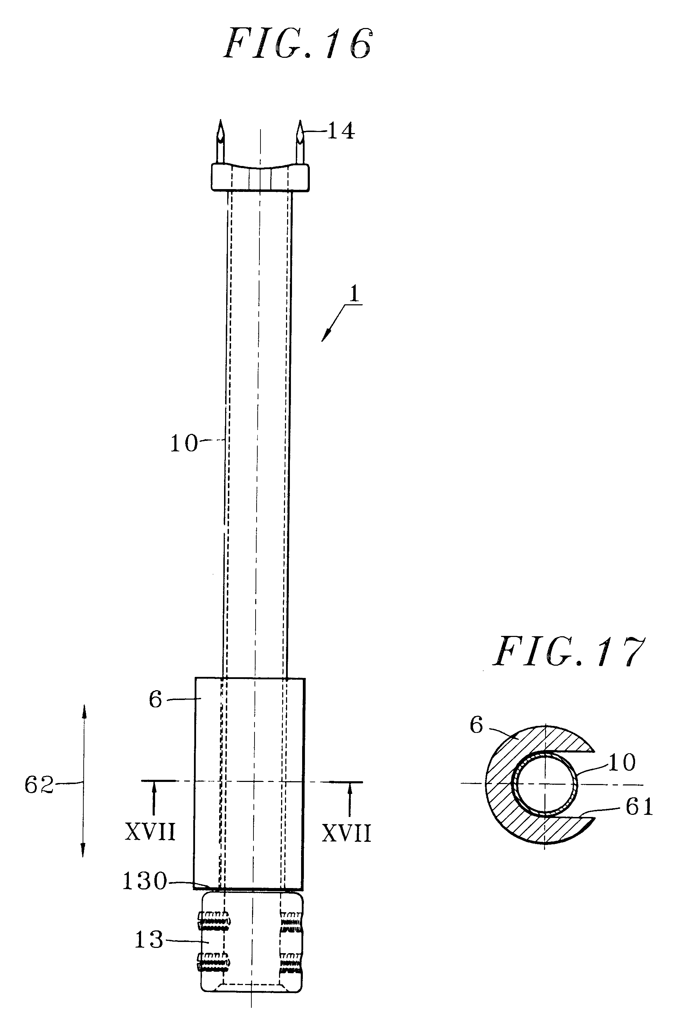

Comme représenté à la figure 14, le tournevis 5 est inséré dans le guide 1 de manière à emmancher l'embout 51 du tournevis 5 dans la tête de vis 361 de la cage 3. En tournant la poignée de manoeuvre 53, le chirurgien visse la vis 36 dans le galet d'écartement 37, ce qui rapproche le galet d'écartement 37 de la tête de vis 361 et en conséquence écarte les deux branches 31 et 32 de la cage suivant un angle de quelques degrés, comme montré à la figure 15. L'élargissement 341 de la fente 34 et donc le rétrécissement de l'épaisseur des deux branches 31 et 32 à leur extrémité postérieure sous le pont 33 forment deux "charnières élastiques" opposées et favorisent une légère flexion des branches 31 et 32 et en conséquence leur écartement. Pour autoriser ces flexions, les dentures postérieures 312 et 322 ont des dents plus petites que celles des dentures antérieures 313 et 323. Lorsque les branches ont l'écartement souhaité, le chirurgien retire le tournevis 5 du guide 1. L'écartement angulaire des deux branches de la cage permet d'adapter la position relative des deux vertèbres suivant l'angle de lordose dans le plan antéropostérieur. L'écartement maximum correspond environ à une augmentation de 2 à 3 mm de l'épaisseur de la cage 3 à son extrémité antérieure écartée.

L'écartement des deux branches est ainsi symétrique par rapport à l'axe P-A dans le plan de la figure 15. En variante, les surfaces d'appui 310 et 320, et/ou l'élargissement 341 et/ou le galet ou élément d'écartement 37 sont dissymétriques, de sorte que l'une des branches 31 et 32 subit une flexion supérieure à celle de l'autre branche par rapport à l'axe P-A lorsque la vis 36 est vissée dans le galet ou élément d'écartement 37.

En référence aux figures 16 et 17, lorsque l'implantation et le réglage de la cage 3 sont terminés, le guide est retiré à l'aide d'une enclume 6 sous la forme d'une pièce cylindrique métallique dans laquelle a été usinée une rainure longitudinale 61. La largeur de la rainure 61 est sensiblement supérieure au diamètre externe du corps 10 de guide 1 et inférieure au diamètre de l'extrémité distale 13 du guide. L'enclume 6 a ainsi une section transversale en U. Le chirurgien enfile l'enclume 6 sur le corps 10 du guide et applique de légères poussées, si besoin est avec un marteau, contre la surface d'appui 130 de l'extrémité 13 du guide, comme montrée par la flèche 62, pour dégager les pointes 14 du milieu osseux vertébral dans lequel elles étaient précédemment plantées.

La cage intersomatique 3 est réalisable en plusieurs tailles pour une bonne adaptation anatomique au patient et le matériel ancillaire dont les dimensions sont fonction de celles de la cage est en conséquence également réalisable en plusieurs tailles. A titre d'exemple, la cage 3 peut avoir une section carrée de côté de 9 ou 11 ou 13 mm.

En pratique, deux cages selon l'invention sont juxtaposées dans un même espace intervertébral ; les cages sont à une distance moyenne de 10 à 15 mm environ l'une de l'autre, et convergent sensiblement de 10 à 15° environ vers la direction antérieure.

## Revendications

**1 -** Cage intersomatique (3) à insérer entre deux corps vertébraux (CV1, CV2) caractérisée en ce qu'elle comprend deux branches sensiblement parallèles (31, 32) pour être introduites entre lesdits corps vertébraux, un pont (33) reliant des premières extrémités des branches, et un moyen (36, 37) pour écarter angulairement des secondes extrémités des branches (31, 32) après insertion de la cage (3) entre les corps vertébraux.

**2 -** Cage conforme à la revendication 1, caractérisée en ce que les secondes extrémités des branches (31, 32) ont des surfaces d'appui en regard (310, 320) qui forment les côtés d'un vé dont le sommet est orienté vers le pont (33) et qui sont en contact glissant avec le moyen pour écarter (36, 37).

**3 -** Cage conforme à la revendication 1 ou 2, caractérisée par une fente (34) s'étendant depuis les secondes extrémités des branches (31, 32), située entre les branches et traversée longitudinalement par le moyen pour écarter (36, 37), ladite fente étant de préférence terminée près du pont (33) par un élargissement (341).

**4 -** Cage conforme à l'une quelconque des revendications 1 à 3, caractérisée en ce que le moyen pour écarter comprend
une vis (36) comprenant une tige filetée (362) traversant le pont et passant entre les branches (31, 32), et
une pièce d'écartement (37) en contact avec des surfaces d'appui en regard (310, 320) des secondes extrémités de branche et vissée à la tige filetée de la vis.

**5 -** Cage conforme à la revendication 4, caractérisée en ce que l'extrémité de la tige filetée (362) de la vis (36) comprend un moyen d'arrêt (363) pour maintenir la pièce d'écartement (37) sur la tige filetée.

**6 -** Cage conforme à la revendication 4 ou 5, caractérisée en ce que la tête de la vis (36) comprend un moyen (361) pour être manoeuvré par un tournevis.

**7 -** Cage conforme à l'une quelconque des revendications 1 à 6, caractérisée en ce qu'une face du pont (33) opposée aux branches (31, 32) comprend un logement circulaire (333) parallèle aux branches et traversé perpendiculairement par une rainure (334) pour introduire un tenon d'extrémité correspondant (411) d'un porte-cage (4).

**8 -** Cage conforme à l'une quelconque des revendications 1 à 7, caractérisée en ce qu'elle est sensiblement parallélépipédique.

**9 -** Cage conforme à l'une quelconque des revendications 1 à 8, caractérisée en ce que des faces externes (311, 321) des branches (31, 32) comprennent des moyens d'ancrage transversaux (312, 313 ; 322, 323), de préférence répartis en des dentures plus petites (312, 322) aux premières extrémités de branche et des dentures plus grandes (313, 323) aux secondes extrémités de branche.

**10 -** Cage conforme à l'une quelconque des revendications 1 à 9, caractérisée en ce que les branches (31, 32) comprennent des trous oblongs (35) s'étendant sensiblement entre les premières et secondes extrémités des branches respectivement.

**11 -** Guide (1) pour guider une cage (3) conforme à l'une quelconque des revendications 1 à 10, vers un milieu osseux tel qu'espace intervertébral entre deux corps vertébraux (CV1, CV2), caractérisé en ce qu'il comprend un conduit longitudinal de guidage (11) dans lequel la cage (3) peut glisser longitudinalement, et deux pointes saillantes (14) devant une première extrémité (12) du conduit de guidage pour être plantées dans le milieu osseux.

**12 -** Guide conforme à la revendication 11, caractérisé en ce que le conduit de guidage (11) est cylindrique et comprend à une seconde extrémité (13) un moyen (133, 134) pour positionner en rotation la cage (3) introduite par la seconde extrémité (13).

**13 -** Guide conforme à la revendication 12, caractérisé en ce que le moyen pour positionner comprend un ergot (133 ; 134) saillant radialement vers l'intérieur du conduit de guidage (11).

**14 -** Guide conforme à l'une quelconque des revendications 11 à 13, comprenant une surface d'appui (130) située sensiblement à une seconde extrémité (13) du conduit de guidage (11), sensiblement perpendiculaire au conduit de guidage (11), et dirigée vers la première extrémité (12).

**15 -** Râpe (2) pour façonner un logement notamment entre deux corps vertébraux (CV1, CV2) pour y insérer une cage (3) conforme à l'une quelconque des revendications 1 à 10, caractérisée en ce qu'elle comprend une tête de râpe (20) ayant une section transversale analogue à la section transversale de la cage (3) perpendiculaire aux branches, et un conduit de râpe longitudinal (23) débouchant en bout de la tête de râpe, au moins une face longitudinale (25, 26) de la tête de râpe étant munie de dents (27) et d'encoches (24), lesdites encôches étant localisées entre les dents et débouchant dans le conduit de râpe (23).

**16 -** Râpe conforme à la revendication 15, caractérisée en ce que les encoches (24) sont inclinées d'environ 30 à 45° par rapport à ladite face longitudinale (25, 26) de la tête de râpe.

**17 -** Râpe conforme à la revendication 15 ou 16, caractérisée en ce qu'elle comprend une rainure longitudinale (221) pour coopérer avec le moyen pour positionner en rotation (133, 134) dans le guide (1) conforme à la revendication 12 ou 13.

**18 -** Râpe conforme à l'une quelconque des revendications 15 à 17, comprenant une butée externe (223) localisée à une autre extrémité (22) de la râpe opposée à la tête de râpe (20) pour buter contre une seconde extrémité (13) du guide (1) conforme à l'une quelconque des revendications 11 à 14.

**19 -** Porte-cage (4) pour liaison amovible avec la cage (3) conforme à la revendication 7, caractérisé en ce qu'il comprend un tenon d'extrémité (41) ayant une section transversale sensiblement complémentaire du logement circulaire (333) dans le pont de cage (33) et susceptible de traverser librement la rainure (334) dans le pont de cage.

**20 -** Porte-cage conforme à la revendication 19, comprenant un corps (42) ayant une rainure longitudinale (422) pour coopérer avec le moyen pour positionner en rotation (133, 134) du guide (1) conforme à la revendication 12 ou 13, et un dégagement (421) situé à une extrémité de la rainure (422) opposée au tenon, le dégagement, lorsqu'il est en regard du moyen pour positionner en rotation (133, 134), autorisant une rotation axiale du porte-cage dans le conduit de guidage (11).

**21 -** Tournevis (5) pour la cage conforme à l'une quelconque des revendications 4 à 6, caractérisé par un corps (52) pouvant être introduit dans le conduit de guidage (11) du guide (1) conforme à l'une quelconque des revendications 11 à 14, et ayant à une extrémité (521) un embout (51) à section complémentaire d'un moyen dans la tête de vis (361) pour tourner la tête de vis, et à une autre extrémité une poignée de manoeuvre (53).
